# EUROPEAN PATENT APPLICATION

(11) **EP 4 070 741 A1**
(43) Date of publication of application: **12.10.2022**
(21) Application number: 20897311.5
(22) Date of filing: 20.11.2020
(51) Int. Cl.: A61B 17/15, A61B 17/56

(54) **INSTRUMENT FOR BONE SURGERY**

(30) Priority: 05.12.2019 JP 2019220047
(71) Applicant: Olympus Terumo Biomaterials Corp., Tokyo 151-0073 (JP)
(72) Inventor: AKIYAMA, Takenori, Kasuya-gun, Fukuoka 811-0119 (JP); YOKOYAMA, Yasuharu, Tokyo 151-0073 (JP)
(74) Representative: Schicker, Silvia
(86) International application number: PCT/JP2020/043425
(87) International publication number: WO 2021/111907

(57) **Abstract**

A highly versatile bone surgery instrument that enables performing an arc osteotomy precisely and easily. A bone surgery instrument 1 includes a center portion 2 to be positioned at a specific point in a bone, a guide portion 3 including a slit 4 extending in a circumferential direction around an axis line I extending through the center portion 2, the slit 4 which is extending through the guide portion 3 in a direction parallel to the axis line I and which guides an osteotome blade inserted in the slit 4, in the circumferential direction around the axis line I, and a fixing portion 6 to be fixed to the bone using a fixing member.

## Description

### {Technical Field}

The present invention relates to a bone surgery instrument and, specifically, relates to an instrument that is used as a guide for guiding an osteotome blade in an osteotomy.

### {Background Art}

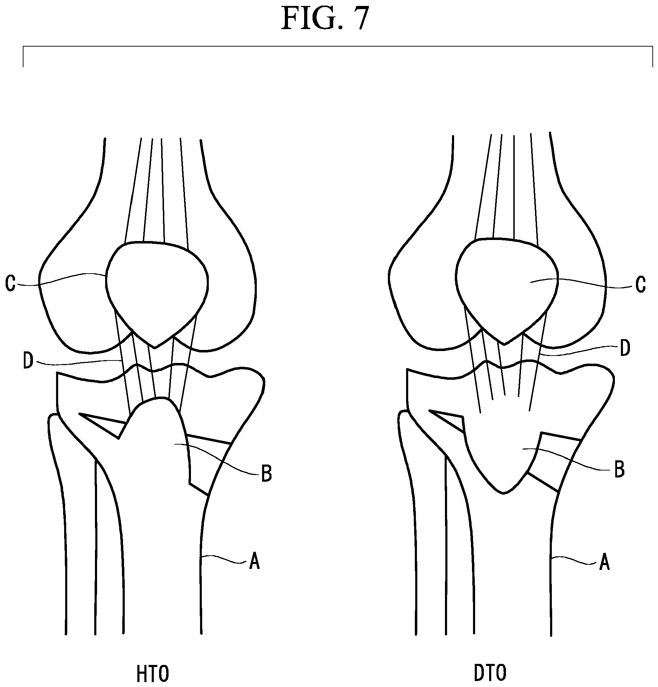

Conventionally, as treatment of joint pain on the inner side of a knee, opening wedge high tibial osteotomy (HTO) and opening wedge distal tuberosity osteotomy (DTO), in which knee alignment is corrected, have been known. As illustrated in Fig. 7, in HTO, an osteotomy is performed on the proximal side of a tuberosity part B, and in DTO, an osteotomy is performed on the distal side of a tuberosity part B.

In HTO, there is a problem of osteoarthritis (OA) of the patellofemoral joint (PF) of a knee due to lowering of the height of the kneecap C. Unlike HTO, DTO causes no change in position of the kneecap C between before and after the surgery. Therefore, in comparison with HTO, DTO has the advantageous of being unlikely to cause progression of PFOA due to lowering of the height of the kneecap C after surgery. On the other hand, DTO has a point that is to be improved and that is related to instability of the bone after surgery due to the epiphyseal region of the tibia A being pulled by the patellar tendon D and that is related to the resulting delay in bone union.

As another method of tibial osteotomy, a method in which a tibia is osteotomized in an arc-like shape has been proposed (see, for example, NPL 1). The arc-like bone cutting increases the contact area of the osteotomized portion and the leads to expectation of early bone union.

Furthermore, as another method of tibial osteotomy, DTAO (distal tibia tuberosity arc osteotomy) in which a distal portion of a tuberosity part is osteotomized in an arc-like shape has been proposed. Unlike the method of NPL 1 in which a part, on the distal side relative to the tuberosity part, of a tibia is separated, in DTAO, the distal portion of the tuberosity part is osteotomized to an intermediate point in an anteroposterior direction so that continuity between the epiphyseal
region and the diaphyseal region of the tibia is maintained.

### {Citation List}

### {Non Patent Literature}

### {NPL 1}

Kenichi Tagaya and three others, "The Effects of High Tibial Osteotomies, Using Citieffe Curvilinear Osteotome Blades, on Bone Unions", The Journal of the Chugoku-Shikoku Orthopaedic Association, The Chugoku-Shikoku Orthopaedic Association, April 15, 2000, Volume 12, Issue 1, p. 111-116.

### {Summary of Invention}

### {Technical Problem}

One of points to be improved of the method of NPL 1 is high complexity of osteotomization operation. In NPL 1, a tibia is osteotomized in an arc-like shape with a curvilinear osteotome blade while the curvilinear osteotome blade is knocked with a hammer. In this method, where the vicinity of a tuberosity is osteotomized in an arc-like shape in DTAO, since the cortical bone below the tuberosity is hard, it is difficult to precisely osteotomize the tuberosity part along an arc-like line, and a load is applied to the bone because of the curvilinear osteotome blade being knocked, resulting in difficulty in maintenance of continuity of the bone. If bumps and dips occur in the osteotomized surface of the tuberosity part, the contact area decreases and a load is unevenly applied to the bone because of the osteotomized surface being forcibly brought into contact with the adjacent bone surface, which may cause an increase in instability of the bone.

The present invention has been described in view of the aforementioned circumstances and an object of the present invention is to provide a versatile bone surgery instrument enabling performing an arc osteotomy precisely and easily.

### {Solution to Problem}

In order to achieve the above object, the present invention provides the following solution.

An aspect of the present invention provides a bone surgery instrument for guiding an osteotome blade in a circumferential direction around a specific point in a bone, the bone surgery instrument including: a center portion to be positioned at the specific point; a guide portion including a slit extending in a circumferential direction around an axis line extending through the center portion, the slit which is extending through the guide portion in a direction parallel to the axis line and which guides the osteotome blade inserted in the slit in the circumferential direction around the axis line; and a fixing portion to be fixed to the bone using a fixing member.

In the present aspect, the center portion is positioned at a specific point of a bone, the guide portion is disposed on the bone in such a manner that the slit is located at a position to be subjected to osteotomization, and the fixing portion is fixed to the bone using the fixing member to prevent the bone surgery instrument from moving relative to the bone. In this state, the slit extends in the circumferential direction around the specific point. Therefore, the bone can be osteotomized in an arc-like or substantially arc-like shape precisely and easily by advancing the osteotomization using the osteotome blade while moving the osteotome blade according to guiding via the slit.

Also, the bone surgery instrument can be used with any of various osteotome blades each having a thickness that is smaller than a width of the slit, and thus, a versatile bone surgery instrument can be provided.

In the above aspect, the bone surgery instrument may be used for osteotomizing a distal portion of a tuberosity part of a tibia in an arc-like or substantially arc-like shape.

In other words, positioning the center portion at a hinge point of an epiphyseal region of a tibia enables a distal portion of a tuberosity part to be osteotomized precisely and easily along an arc-like or substantially arc-like line around the hinge point as a center.

In the above aspect, the guide portion may have a thickness that makes an amount of projection of a tip of the osteotome blade from the slit be equal to or below a predetermined amount.

The amount of projection of the distal end tip of the osteotome blade from the slit is limited by the thickness of the guide portion. The above configuration enables preventing excessive osteotomization in the direction parallel to the axis line by limiting the amount of projection of the tip from the slit to be equal to or below the predetermined amount.

In the above aspect, the center portion may include a pin hole extending through the center portion in a direction parallel to the axis line.

In an osteotomy, a pin, which serves as a positional and directional reference may be inserted in a specific point of a bone. The center portion is positioned at the specific point by the pin extending through the inside of the pin hole and a depth direction of the slit is parallel to a direction of a longitudinal axis of the pin. Therefore, an osteotomized surface parallel to the longitudinal axis of the pin can be formed.

In the above aspect, the fixing portion may be provided at an opposite side of the center portion relative to the guide portion.

This configuration enables, when osteotomization is performed along a plane intersecting with the axis line extending through the center portion, using a cutting instrument, with the bone surgery instrument disposed on the bone, preventing interference with a fixing member for the cutting instrument.

In the above aspect, the width of the slit may be larger than a thickness of the osteotome blade by 0.5 mm to 1.5 mm.

With this configuration, in a state in which the osteotome blade is inserted in the slit, an adequate space is formed inside the slit. Consequently, it is possible to prevent interference of the osteotome blade with an inner surface of the slit while reducing shaking of the osteotome blade during cutting.

In the above aspect, in a plan view seen along a direction of the axis line, an outer shape of the guide portion may be a similar figure or a substantially similar figure of a shape of the slit, and a thickness of an edge portion of the guide portion, the edge portion surrounding the slit, may be 1 mm to 5 mm.

This configuration enables accurately grasping a position of the slit from an image of the guide portion in X-ray fluoroscopy. Therefore, the configuration enables, in an osteotomy in which a bone is osteotomized to the position of the slit along the plane intersecting with the axis line extending through the center portion, preventing excessively osteotomizing the bone beyond the position of the slit.

In the above aspect, a contact surface on one side in the direction along the axis line, the contact surface being in contact with a surface of the bone, may be included, and the contact surface may be a concave surface adapted to a shape of the surface of the bone.

A surface of the bone on which the bone surgery instrument is disposed is a convex curve surface. The above configuration enables the bone surgery instrument to be more stably positioned relative to the bone, by disposing the bone surgery instrument on the bone in such a manner that the concave surface comes into contact with the surface of the bone, and enables reduction of shaking of the bone surgery instrument during osteotomization.

In the above aspect, a support rod portion that interconnects the center portion and the guide portion may be included, and the support rod portion may extend in a direction intersecting with the axis line, between the center portion and a center in the circumferential direction of the guide portion.

With this configuration, in the plan view in the direction along the axis line extending through the center portion, the bone surgery instrument has a substantially T-shape that is symmetrical with respect to the support rod portion. Such shape prevents inclination of the bone surgery instrument disposed on the bone, enabling the bone surgery instrument to be more stably disposed relative to the bone.

In the above aspect, the slit may extend along an arc having a constant curvature radius.

After osteotomization according to guiding via the arc-like slit, a gap according to the thickness of the osteotome blade is formed along an arc-like osteotomization line. With the above configuration, a distance between the specific point and the osteotomization line is the same over an entire length of the osteotomization line. Therefore, it is possible to rotate a bone piece including the specific point around the specific point while a width of the gap being maintained constant. In other words, it is possible to easily rotate the bone piece without two bone surfaces facing each other across the gap interfering with each other during the rotation.

In the above aspect, the slit may extend along a curve having a curvature radius that gradually decreases in the circumferential direction around the axis line.

With this configuration, a distance between the center portion and the slit gradually decreases in the circumferential direction from one end toward another end of the slit, and thus, the distance between the specific point and the osteotomization line also gradually decreases from one end toward another end of the osteotomization line.

Therefore, as the bone piece including the specific point is rotated around the specific point, the gap gradually narrows. Consequently, compression and deformation of the bone can be reduced, the compression and deformation being necessary for bringing the two bone surfaces facing each other across the gap into contact with each other after correction of the bone by rotation of the bone piece.

In the above aspect, the slit may include a plurality of linear portions that are continuous with each other in the circumferential direction around the axis line and each of the plurality of linear portions may extend in a direction tangent or substantially tangent to a circle whose center is the axis line.

This configuration enables even an osteotome blade of a type that is movable on a parallel surface to be easily guided by the slit.

In the above aspect, preferably, each of triangles whose vertices are opposite ends of each of the linear portions and the center portion is a right triangle whose base is the linear portion; and an opposite side of one of the right triangles and a hypotenuse of another one of the right triangles adjacent to the one of the right triangles are shared with each other.

This configuration enables easily designing a shape of the slit including a plurality of linear portions and having a curvature radius that gradually decreases, using a plurality of right triangles aligned continuously in the circumferential direction around the center portion.

In the above aspect, a distance between the center portion and the slit may decrease by a predetermined length, for each position shifted by a predetermined angle in the circumferential direction around the axis line.

With this configuration, each time the bone piece including the specific point is rotated by the predetermined angle, the gap narrows by the predetermined length. Therefore, designing the predetermined angle and the predetermined length according to a correction angle of the bone piece including the specific point and the thickness of the osteotome blade enables easily making a design such that the width of the gap after rotation becomes a desired dimension.

In the above aspect, a distance between the center portion and the slit may be 45 mm to 65 mm.

This configuration enables, in osteotomization of a distal portion of a tuberosity part, preventing interference of an arc-like or substantially arc-like osteotomization line with the patellar tendon. Also, the configuration enables ensuring that an osteotomized portion of the tuberosity part, the osteotomized portion being located on the proximal side relative to the osteotomization line, is sufficiently wide, and thus enables inserting bone screws for fixing the osteotomized portion, to the osteotomized portion.

In the aspect, a movable portion joined to the guide portion in such a manner as to be movable in the circumferential direction around the axis line may be included, and the movable portion may include a guide hole extending through the movable portion in a direction parallel to a radial direction orthogonal to the axis line and guiding a guide wire or a guide sleeve to which the guide wire is inserted, toward the axis line, and an outer circumferential surface of the guide wire extending through the guide hole may be disposed at a position distant from the axis line in an offset direction orthogonal to the axis line and a center axis line of the guide hole.

In the aspect, the movable portion may include a second guide portion that guides the osteotome blade along a plane orthogonal to the axis line.

### {Advantageous Effects of Invention}

The present invention exerts an effect of enabling provision of a highly versatile bone surgery instrument enabling performing an arc osteotomy precisely and easily.

### {Brief Description of Drawings}

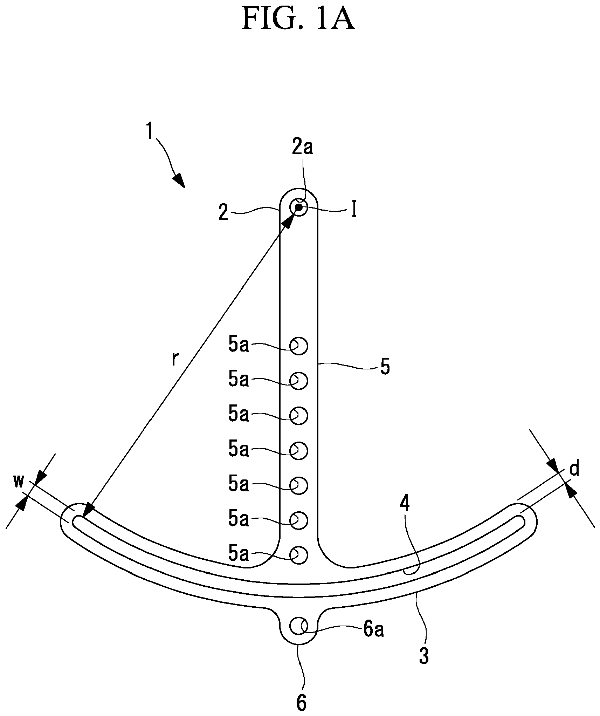

{Fig. 1A}
   Fig. 1A is a plan view of a bone surgery instrument according to an embodiment of the present invention.
{Fig. 1B}
   Fig. 1B is a side view of the bone surgery instrument in Fig. 1A.
{Fig. 2}
   Fig. 2 is a diagram illustrating a procedure of DTAO using the bone surgery instrument in Figs. 1A and 1B.
{Fig. 3}
   Fig. 3 is a diagram illustrating a procedure of DTAO using the bone surgery instrument in Figs. 1A and 1B.
{Fig. 4}

   Fig. 4 is a diagram illustrating a procedure of DTAO using the bone surgery instrument in Figs. 1A and 1B.
{Fig. 5}
   Fig. 5 is a perspective view of a modification of the bone surgery instrument in Fig. 1A.
{Fig. 6}
   Fig. 6 is a plan view of another modification of the bone surgery instrument in Fig. 1A.
{Fig. 7}
   Fig. 7 is a diagram illustrating HTO and DTO.
{Fig. 8A}
   Fig. 8A is a plan view of a bone surgery instrument according to another embodiment of the present invention.
{Fig. 8B}
   Fig. 8B is a side view of the bone surgery instrument in Fig. 8A in a direction along a center axis line of a guide hole.
{Fig. 8C}
   Fig. 8C is a perspective view of the bone surgery instrument in Fig. 8A and illustrates an example of a joining structure that joins a movable portion to a guide portion.
{Fig. 9A}
   Fig. 9A is a plan view of a bone surgery instrument according to another embodiment of the present invention.
{Fig. 9B}
   Fig. 9B is a perspective view of an example of a second part of a movable portion in the bone surgery instrument in Fig. 9A.
{Fig. 9C}
   Fig. 9C is a perspective view of another example of the second part of the movable portion in the bone surgery instrument in Fig. 9A.
{Fig. 9D}
   Fig. 9D is a perspective view of another example of the second part of the movable portion in the bone surgery instrument in Fig. 9A.
{Fig. 10}
   Fig. 10 is a side view illustrating a state in which a guide sleeve and a guide wire are inserted in a guide hole of the second part.

### {Description of Embodiments}

A bone surgery instrument according to an embodiment of the present invention will be described below with reference to the drawings.

A bone surgery instrument 1 according to the present embodiment is used for an osteotomy in which a bone is osteotomized along an arc-like or substantially arc-like line extending in a circumferential direction around a specific point of the bone. In the present embodiment, as an example of osteotomy, DTAO (distal tibia tuberosity arc osteotomy) in which a tuberosity part B of a tibia A is osteotomized in a substantially L-shape will be described. The bone surgery instrument 1 can be used not only in DTAO but also any other osteotomy.

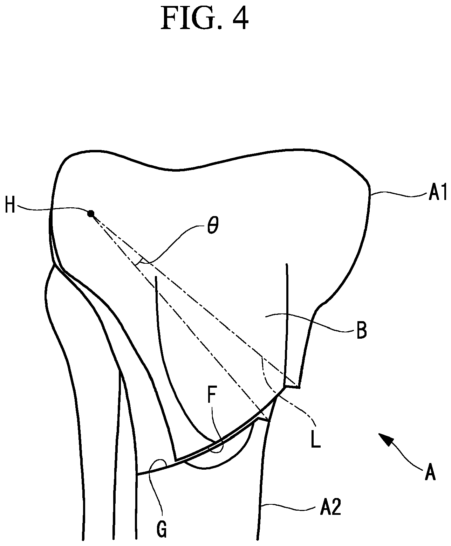

As illustrated in Fig. 4, DTAO is different from opening wedge HTO and DTO, and in the difference, a distal portion of a tuberosity part B is osteotomized along an arc-like or substantially arc-like line around a hinge point H as the center which is used in opening. Fig. 4 illustrates a corrected state resulting from rotation of a group of an epiphyseal region A1 and the osteotomized portion of the tuberosity part B around the hinge point H.

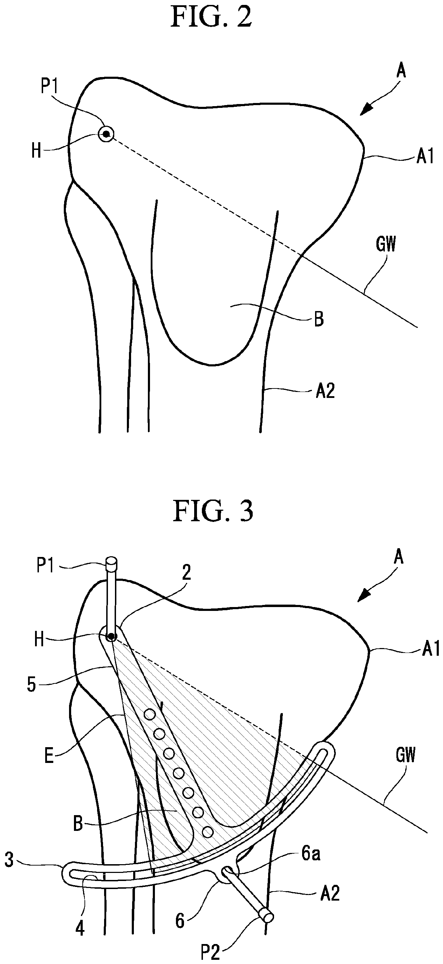

In DTAO, a posterior side of the tuberosity part B is osteotomized in parallel with the tibia axis and the distal portion of the tuberosity part B is osteotomized perpendicularly to the tibia axis. Consequently, as an osteotomized surface, an undersurface E (see Fig. 3) on the posterior side of the tuberosity part B and a side surface F of the distal portion of the tuberosity part B are formed. Here, the distal portion of the tuberosity part B is osteotomized along an arc-like or substantially arc-like line around the hinge point H as the center and the side surface F is a convex surface that curves around an axis line extending through the hinge point H in an anteroposterior direction of the tibia A.

Next, the tibia A is osteotomized from the inner side of the tibia A toward the hinge point H and an osteotomization line L is thus formed.

In the drawings referred to, the right side of the tibia A is the inner side and the left side of the tibia A is the outer side, and a direction perpendicular to the sheet surface is the anteroposterior direction.

Next, the osteotomization line L is opened by the group of the epiphyseal region A1 and the osteotomized portion of the tuberosity part B which are rotated around the hinge point H by a correction angle θ, and deformation of the tibia A is thus corrected. At this time, the side surface F slides along a concave surface G of a diaphyseal region A2.

Next, an artificial bone or an autologous bone is implanted in the opened portion.

Next, with the undersurface E and the side surface F brought into contact with respective adjacent bone surfaces by compression being applied to the osteotomized portion of the tuberosity part B, the osteotomized portion of the tuberosity part B is fixed to the posterior bone A2 via, e.g., bone screws.

In this way, in the case of DTAO, the osteotomized portion of the tuberosity part B are brought into contact with the adjacent bone surfaces at the undersurface E and the side surface F and the number of contact surfaces, and the contact area, of the osteotomized portion increase in comparison with HTO and DTO. Therefore, DTAO is superior in terms of enhancing bone stability and enabling early bone union and fixing strength enhancement of the osteotomized portion.

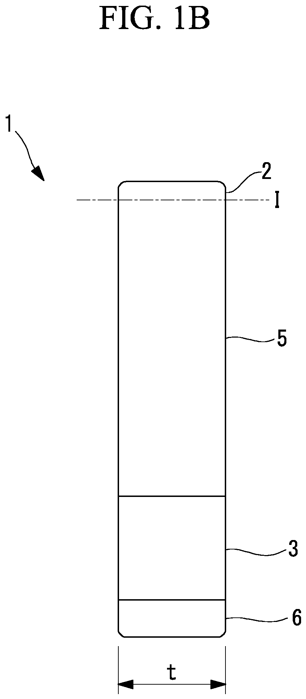

The bone surgery instrument 1 is a guide device that guides an osteotome blade used for formation of the side surface F, along an arc-like or substantially arc-like line. As illustrated in Figs. 1A and 1B, the bone surgery instrument 1 includes a center portion 2, a guide portion 3 including a slit 4, a support rod portion 5 that interconnects the center portion 2 and the guide portion 3, and a fixing portion 6 to be fixed to a tibia A using a fixing member.

Fig. 1A is a plan view of the bone surgery instrument 1 in a direction along a predetermined axis line I extending through the center portion 2 and Fig. 1B is a side view of the bone surgery instrument 1 in Fig. 1A from the right side.

The center portion 2, the guide portion 3, the support rod portion 5 and the fixing portion 6 are integrated, and therefore, the bone surgery instrument 1 is formed of a single member.

It is preferable that the bone surgery instrument 1 be formed of stainless steel, titanium or titanium alloy in consideration of abrasion due to friction with a cutting tool.

The center portion 2 is a part to be positioned at the hinge point (specific point) H of the tibia A, and includes a pin hole 2a extending through the center portion 2 in a direction parallel with the axis line I. As illustrated in Figs. 2 and 3, in DTAO, a hinge pin P1 is inserted in the hinge point H in the anteroposterior direction of the tibia A. The pin hole 2a is coaxial or substantially coaxial with the axis line I and has an inner diameter that is slightly larger than an outer diameter of the hinge pin P1. Inserting the hinge pin P1 into the pin hole 2a enables the center portion 2 to be positioned in the hinge point H in such a manner that the axis line I coincides or substantially coincides with a longitudinal axis of the hinge pin P1.

The guide portion 3 has an arc-like shape extending in a circumferential direction around the axis line I and having a curvature radius that is constant over the entire length. The slit 4 has an arc-like shape extending in the circumferential direction around the axis line I and having a curvature radius that is constant over the entire length. Therefore, in the plan view in the direction along the axis line I, an outer shape of the guide portion 3 is mathematically a similar figure or a substantially similar figure of the shape of the slit 4.

The slit 4 extends through the guide portion 3 in a direction parallel to the axis line I. A thickness t, in the direction parallel to the axis line I, of the guide portion 3, that is, a depth of the slit 4 is smaller than a length of the osteotome blade and a tip of the osteotome blade extending through the slit 4 projects from the slit 4.

A thickness d of an edge portion of the guide portion 3, the edge portion surrounding the slit 4, is substantially constant over an entire circumference. The thickness d is preferably 1 mm to 5 mm from the perspectives of a slim structure of the guide portion 3 and the resulting visibility of the guide portion 3 in a fluoroscopic X-ray image.

A distance r in a radial direction orthogonal to the axis line I between the center portion 2 and the slit 4, that is, a curvature radius of the slit 4 is preferably 45 mm to 65 mm. The distance r corresponds to a distance between the hinge point H and the side surface F. The distance r falling within the aforementioned range enables prevention of interference of an osteotomization line for the side surface F with a patellar tendon D. The distance r falling within the aforementioned range also enables ensuring that the osteotomized portion of the tuberosity part B is sufficiently wide, the osteotomized portion being located on the proximal side relative to the side surface F, and thus enables inserting bone screws for fixing the osteotomized portion, to the osteotomized portion after correction. In a case where the distance r is larger than 65 mm, the osteotomization line for the side surface F becomes close to the diaphyseal region in which the cortical bone is large, resulting in difficulty in osteotomization.

There are individual differences in distance from the hinge point H to the distal portion of the tuberosity part B in which the side surface F is to be formed, among patients. Therefore, it is preferable that bone surgery instruments 1 of a plurality of sizes in which the distance r is different from one another be provided so that a bone surgery instrument 1 of a proper size can be selected for each patient.

The support rod portion 5 extends linearly in the radial direction orthogonal to the axis line I, between the center portion 2 and a center in the circumferential direction of the guide portion 3. Therefore, in the plan view in the direction along the axis line I, the bone surgery instrument 1 has a substantially T-shape that is symmetrical with respect to the support rod portion 5.

The fixing portion 6 includes a fixing hole 6a extending through the fixing portion 6 in a direction parallel with the axis line I. With the center portion 2 positioned at the hinge point H by the hinge pin P1, inserting a fixing pin P2 to the tibia A through the fixing hole 6a enables the bone surgery instrument 1 to be fixed so as not to move relative to the tibia A in a direction orthogonal to the axis line I. The fixing portion 6 is located on the opposite side of the center portion 2 relative to the guide portion 3, and in the case of the bone surgery instrument 1 in Fig. 1A, the fixing portion 6 is located on an extension line of the support rod portion 5.

One or more fixing holes 5a, each of which is similar to the fixing hole 6a, may be provided in the support rod portion 5 so that a surgeon can change or select a position of fixation of the bone surgery instrument 1 via the fixing pin P2. In the example in Fig. 1A, the support rod portion 5 includes seven fixing holes 5a aligned with an interval from one another in a longitudinal direction of the support rod portion 5. Where the plurality of fixing holes 5a are aligned at equal intervals, the plurality of fixing holes 5a can be used as a scale. In other words, after the center portion 2 being positioned at the hinge point H and the bone surgery instrument 1 being disposed on the tibia A, a smaller and proper size of the bone surgery instrument 1 can be selected based on a positional relationship between the distal portion of the tuberosity part B and the plurality of fixing holes 5a.

Dimensions of the guide portion 3 and the slit 4 are designed according to dimensions of the osteotome blade to be used for formation of the side surface F. The osteotome blade is a common osteotome blade like a thin saw blade.

More specifically, the thickness t of the guide portion 3 is designed according to a length of the osteotome blade so that an amount of projection of the tip of the osteotome blade from the slit 4 becomes equal to or below a predetermined amount. For example, the thickness t is preferably larger than the length of the osteotome blade by 5 mm to 25 mm.

Also, a width w of the slit 4 in the radial direction orthogonal to the axis line I is preferably larger than a thickness of the osteotome blade by 0.5 mm to 1.5 mm in order to prevent rattling of the osteotome blade in the slit 4.

In a preferable example, the osteotome blade has a thickness of around 0.3 mm to 0.8 mm, a width of around 5 mm to 15 mm, and a length of around 15 mm to 45 mm. If the thickness and the width of the osteotome blade are excessively large, an amount of bone cut becomes large. As a result, a large compression force is required for bringing the osteotomized surfaces into contact with the adjacent bone surfaces, resulting in increase in load applied to the bone. Also, if the width of the osteotome blade is excessively large, it is difficult to osteotomize the bone in a complicated shape. If the length of the osteotome blade is excessively large, the osteotome blade easily bends because of a load during bone cutting, and if the length of the osteotome blade is excessively small, the bone cannot be cut to a sufficient depth.

In consideration of the dimensions of the osteotome blade above, in order to ensure capability of guiding the osteotome blade via the guide portion 3 and an amount of bone cut, the thickness t of the guide portion 3 is preferably 10 mm to 25 mm. Also, the width w of the slit 4 is preferably 1 mm to 2 mm.

Although the bone surgery instrument 1 in Figs. 1A and 1B has an entirely constant thickness, the center portion 2, the guide portion 3, the support rod portion 5 and the fixing portion 6 may have thicknesses that are different from one another.

Next, an example of DTAO using the bone surgery instrument 1 will be described.

First, as illustrated in Fig. 2, as preparation of osteotomization, a guide wire GW and a hinge pin P1 are inserted to a tibia A. More specifically, a guide wire GW is inserted to a tibia A from the inner side toward a hinge point H. The hinge point H is set near an outer periphery of the tibia A. Two guide wires GW arranged in the anteroposterior direction of the tibia A may be inserted in parallel with each other to the tibia A. Next, a hinge pin P1 is inserted to the hinge point H in an anteroposterior direction of the tibia A.

Next, as illustrated in Fig. 3, the bone surgery instrument 1 is disposed on the tibia A. More specifically, the center portion 2 is positioned at the hinge point H by inserting the hinge pin P1 to the pin hole 2a, and location of the slit 4 at a distal portion of a tuberosity part B is confirmed. If the slit 4 is not located at the distal portion of the tuberosity part B, the size of the bone surgery instrument 1 is changed so that the slit 4 is located at the distal portion of the tuberosity part B. Then, the fixing portion 6 is fixed to the tibia A by inserting a fixing pin P2 to the tibia A in the anteroposterior direction through the fixing hole 6a.

Next, a bone saw is inserted to the tibia A from the inner side and the posterior side of the tuberosity part B is osteotomized in parallel with the tibia axis. In Fig. 3, the hatched area between the guide wire GW and the guide portion 3 is an area to be osteotomized. Consequently, as a first osteotomized surface, an undersurface E is formed between the guide wire GW and the guide portion 3. At this time, a motor-driven or air-driven bone saw may be used.

The osteotomization for the undersurface E is performed while viewing the guide wire GW, the bone surgery instrument 1 and the bone saw in a fluoroscopic X-ray image in the anteroposterior direction of the tibia A. In other words, a surgeon can perform osteotomization using the guide wire GW and the guide portion 3 as marks of boundaries of a range to be subjected to osteotomization. In particular, since the outer shape of the guide portion 3 is mathematically a similar figure or a substantially similar figure of the shape of the slit 4, the surgeon can correctly grasp a position of the slit 4 from the outer shape of the guide portion 3. Therefore, it is possible to prevent excessive osteotomization beyond a side surface F to be formed subsequently.

Also, the fixing portion 6 is located on the opposite side of the center portion 2 relative to the guide portion 3. Therefore, it is possible to, when the posterior side of the tuberosity part B is osteotomized, prevent interference of the bone saw with the fixing pin P2 inserted through the fixing hole 6a.

Next, the distal portion of the tuberosity part B is osteotomized in the anteroposterior direction using the bone surgery instrument 1. In other words, an osteotome blade is inserted to the slit 4 and the tuberosity part B is osteotomized while moving the osteotome blade in a circumferential direction in the slit 4. Consequently, as a second osteotomized surface, the side surface F is formed at the distal portion of the tuberosity part B. The side surface F is a convex surface that curves in an arc-like shape around a longitudinal axis of the hinge pin P1. A gap having a width according to the thickness of the osteotome blade is formed between the side surface F of the tuberosity part B and a concave surface G of an epiphyseal region A1.

Next, the tibia A is osteotomized from the inner side toward the outer side along the guide wire GW. Consequently, a third osteotomized surface is formed along an osteotomization line L.

Next, as illustrated in Fig. 4, the bone surgery instrument 1 is removed from the tibia A and a group of the epiphyseal region A1 and the osteotomized portion of the tuberosity part B is rotated around the hinge point H relative to the diaphyseal region A2 by a correction angle θ. Consequently, alignment of the tibia A is corrected and a load applied to the tibia A is shifted from the inner side to the outer side.

Since the slit 4 has a constant curvature radius, the side surface F and the concave surface G are curve surfaces curving around the hinge point H as a center and each having a constant curvature radius. Therefore, the width of the gap is maintained constant irrespective of an angle of rotation of the osteotomized portion around the hinge point H. In other words, the side surface F slides along the concave surface G while consistently keeping a constant distance to the concave surface G, and interference of the side surface F with the concave surface G is prevented. Consequently, it is possible to easily rotate the osteotomized portion around the hinge point H.

Next, an artificial bone or an autologous bone is implanted in an opened portion on the osteotomization line L.

Next, with the undersurface E and the side surface F brought in contact with respective adjacent bone surfaces by compression being applied to the osteotomized portion in the anteroposterior direction and a direction along the tibia axis, the osteotomized portion is fixed to the posterior bone via, e.g., bone screws.

In this way, according to the present embodiment, in osteotomizing a distal portion of a tuberosity part B, an osteotome blade is guided by the arc-like slit 4 with a center positioned at a hinge point H. Therefore, it is possible to precisely and easily osteotomize the tuberosity part B in an arc-like shape with the hinge point H as a center.

Also, the bone surgery instrument 1 can be combined with any of various osteotome blades each having a thickness that is smaller than the width w of the slit 4, and thus, it is possible to provide a highly versatile bone surgery instrument 1.

Examples of methods for arc osteotomy include a method using an arc-like chisel and a method using a motor-driven bone saw that can move in an arc-like fashion. However, where a chisel is used, an impact resulting from the chisel being knocked with a hammer is applied to the tibia A, which may make maintenance of continuity between the epiphyseal region A1 and the diaphyseal region A2 difficult. Where a motor-driven bone saw is used, it is difficult to make a center of rotation of the bone saw coincide with the hinge point H.

On the other hand, the present embodiment enables osteotomization without excessive load being applied to a tibia A and with the center portion 2, which is a center of rotation of the osteotome blade, made to coincide with the hinge point H.

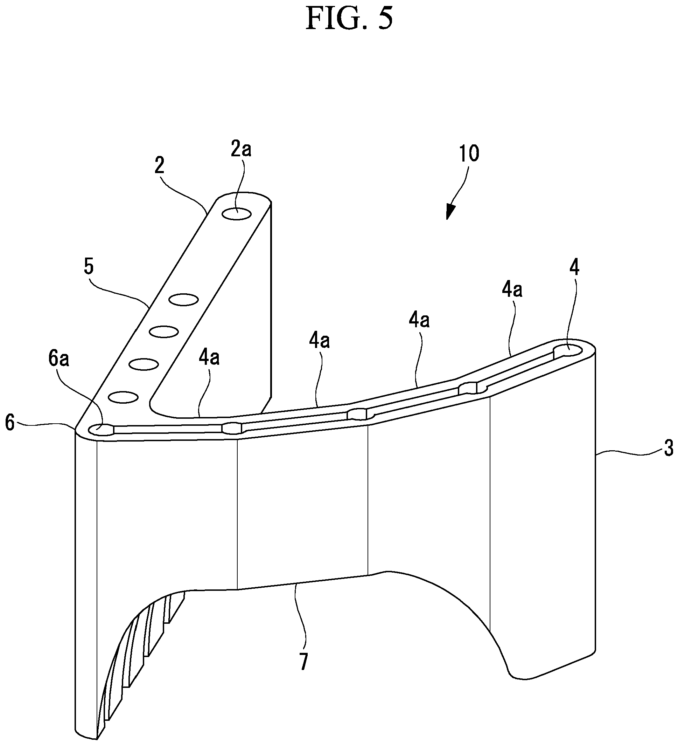

In the present embodiment, as illustrated in Fig. 1B, a surface of the bone surgery instrument 1, the surface being in contact with a surface of the tibia A, may be a flat surface, but as illustrated in Fig. 5, may be a concave surface (contact surface) 7 that is adapted to a shape of the surface of the tibia A. In a bone surgery instrument 10 in Fig. 5, a fixing portion 6 is provided at a connection part of a guide portion 3 and a support rod portion 5.

A concave surface 7 is formed at an end surface, on one side in a direction along an axis line I, of the bone surgery instrument 10, for example, at an end surface of the guide portion 3. The anterior surface of a tibia A on which the bone surgery instrument 10 is disposed is a convex curve surface. As a result of the bone surgery instrument 10 being disposed on the tibia A in such a manner that the concave surface 7 extends along the surface of the tibia A, a position and a posture of the bone surgery instrument 10 relative to the tibia A can be more stabilized, enabling reduction of displacement of the bone surgery instrument 10 during osteotomization.

In the present embodiment, the slit 4 extends along an arc having a constant curvature radius; however, instead, the slit 4 may extend along a curve in which a curvature radius, that is, the distance r gradually decreases from one end toward another end.

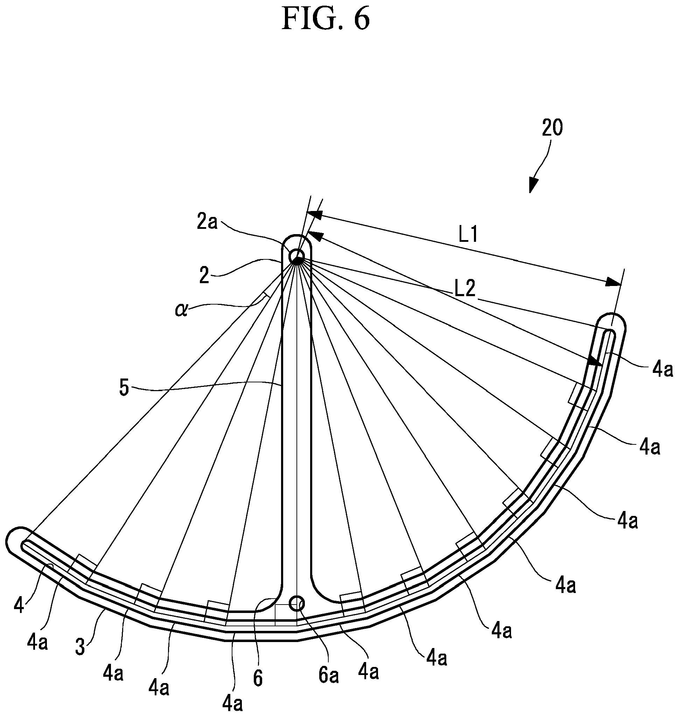

Also, in the present embodiment, the slit 4 smoothly curves over the entire length; however, instead, as illustrated in Figs. 5 and 6, the slit 4 may be formed of a plurality of linear portions 4a that are continuous with one another in a circumferential direction and each extend in a direction tangent or substantially tangent to a circle with an axis line I as the center.

In the bone surgery instrument 20 in Fig. 6, a slit 4 is formed of a plurality of linear portions 4a, and a distance r between a center portion 2 and the slit 4 gradually decreases from one end toward another end of the slit 4. In the case of the bone surgery instrument 20 for DTAO, one end of the slit 4 is disposed on the outer side of a tibia A and the other end of the slit 4 is disposed on the inner side of the tibia A.

As illustrated in Fig. 6, a triangle with opposite ends of each linear portion 4a and the center portion 2 may be a right triangle with a linear portion 4a as the base. Each right triangle includes a hypotenuse, and a base and an opposite side forming a right angle therebetween, and the hypotenuse and the opposite side extend in a radial direction from the axis line I. The opposite side of one right triangle and the hypotenuse of another right triangle adjacent to the one right triangle are shared with each other and have lengths that are equal to each other. Therefore, the slit 4 is formed from the bases of a plurality of right triangles aligned continuously around the axis line I. In each right triangle, a length L1 of the opposite side is smaller than a length L2 of the hypotenuse, and thus, in Fig. 6, a length of a side of the right triangle, that is, the distance r gradually decreases from the left side toward the right side.

Upon the osteotome blade being guided along such slit 4, osteotomized surfaces F, G in which a distance from the hinge point H gradually decreases from the outer side toward the inner side of the tibia A are formed. Therefore, as an osteotomized portion rotates to the inner side during correction, a gap between the side surface F and the concave surface G becomes narrower. In other words, at an initial time of the rotation, there is a sufficient gap between the side surface F and the concave surface G, and thus, the osteotomized portion can be rotated without interfering with the concave surface G. Furthermore, at an end of the rotation, the gap is narrowed, only small compression and deformation of the osteotomized portion are necessary for bringing the side surface F into contact with the concave surface G, enabling reduction of a load on the tibia A.

Also, where apex angles α of the plurality of right triangles in the center portion 2 are equal to one another, a width of the gap after correction is uniform over an entire length. Therefore, the side surface F and the concave surface G can be brought into uniform contact with each other by compression in a direction along the tibia axis, enabling preventing a load from concentrating on a part of the tibia A after correction.

Also, as a result of the slit 4 being formed of the linear portions 4a, where an osteotome blade that moves on a parallel plane is used, interference between the osteotome blade and an inner surface of the slit 4 can be prevented.

In another example of the slit 4 in which the distance r gradually decreases in the circumferential direction, a slit 4 may be designed in such a manner that a distance r between a center portion 2 and the slit 4 decreases by a predetermined length Δr, for each position shifted by a predetermined angle θ' in a circumferential direction around an axis line I.

In this case, when an osteotomized portion is rotated around a hinge point H by the predetermined angle θ', the width of the gap narrows by the length Δr. Therefore, if the predetermined angle θ' is equal to a correction angle θ and the length Δr is equal to the width of the gap between the side surface F and the concave surface G, the gap resulting from osteotomization, after rotation of the osteotomized portion by the correction angle θ, the side surface F and the concave surface G come into contact with each other and the gap is thus eliminated. In other words, after the correction, the side surface F can be brought into contact with the concave surface G with no load applied to the tibia A.

In the above embodiment, the support rod portion 5 interconnects the center portion 2 and the center of the guide portion 3; however, instead, the support rod portion 5 may interconnect the center portion 2 and another position in the guide portion 3. For example, as illustrated in Fig. 5, the support rod portion 5 may interconnect the center portion 2 and one end portion of the guide portion 3.

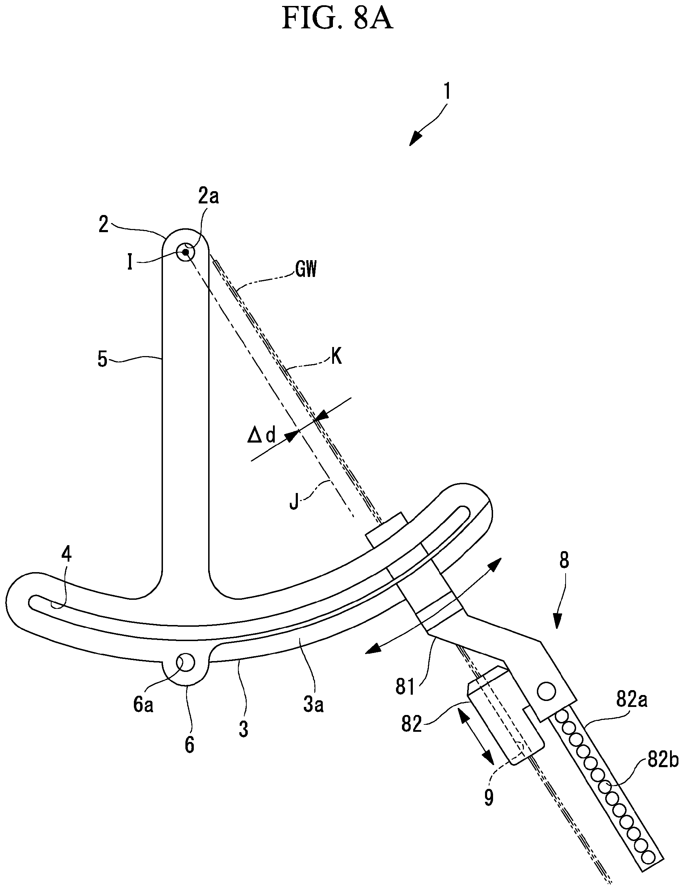

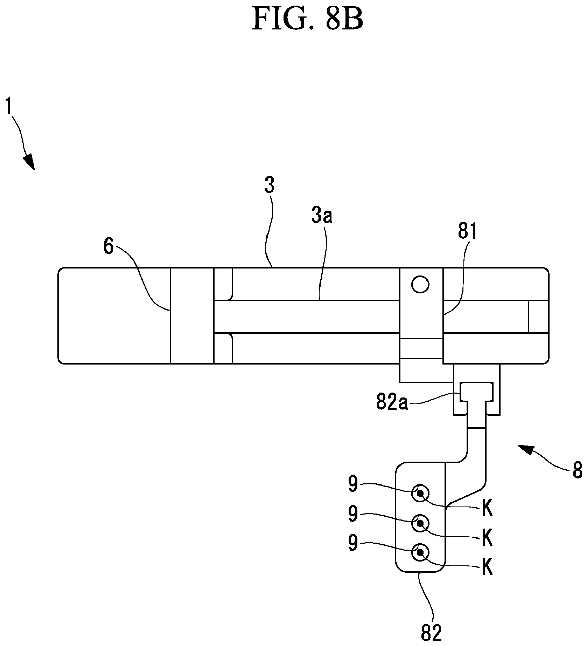

In the above embodiment, as illustrated in Figs. 8A to 8C, the bone surgery instrument 1 may further include a movable portion 8 joined to a guide portion 3 in such a manner as to be movable in a circumferential direction around an axis line I. The movable portion 8 is a component for guiding a guide wire GW in such a manner that the guide wire GW is disposed in a predetermined positional relationship with a hinge pin P1 inserted in a pin hole 2a. The movable portion 8 is applicable also to the bone surgery instruments 10, 20.

The movable portion 8 includes a first part 81 joined to the guide portion 3 and a second part 82 joined to the first part 81.

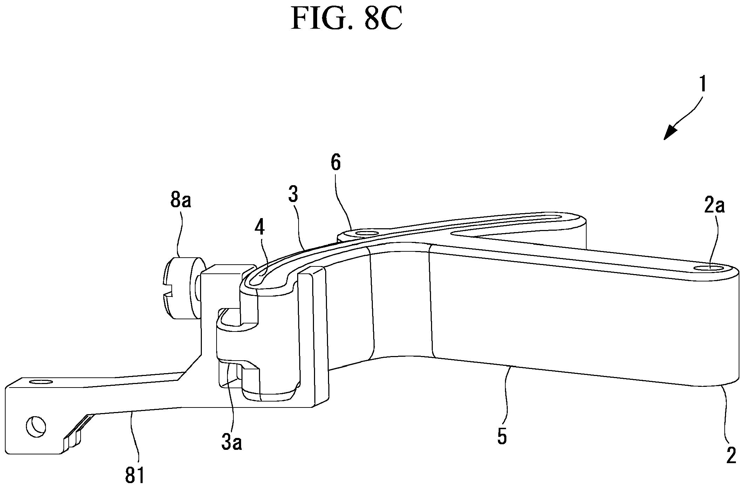

The first part 81 is movable in a circumferential direction along an outer surface of the guide portion 3. Fig. 8C illustrates an example of a joining structure that joins the first part 81 to the guide portion 3. In Fig. 8C, the guide portion 3 includes a rail portion 3a on an outer surface on the radially outer side, the rail portion 3a projecting radially outward. The first part 81 is a substantially C-shape annular member surrounding the guide portion 3 and moves along the rail portion 3a. Where the rail portion 3a is provided at a center in a thickness direction of the guide portion 3, the bone surgery instrument 1 can be used for either of the right and left legs by attaching the first part 81 to the guide portion 3 upside down. The movable portion 8 may include a screw 8a for temporarily fixing the first part 81 to the guide portion 3.

The joining structure is not limited to the above-described structure and may arbitrarily be changed as long as a position of the first part 81 relative to the guide portion 3 in the up-down direction and the radial direction is stabilized and the first part 81 is movable in the circumferential direction relative to the guide portion 3. The up-down direction is a direction parallel to the axis line I.

The second part 82 is disposed on the opposite side of the center portion 2 relative to the guide portion 3. The second part 82 includes at least one guide hole 9 that guides the guide wire GW toward the axis line I side in a direction parallel to a radial direction. The guide hole 9 is located on one side (lower side) in the up-down direction relative to the guide portion 3 and extends through the second part 82 in the direction parallel to the radial direction. A plurality of guide holes 9 may be aligned in a row in the up-down direction so that a plurality of guide wires GW can be inserted to a tibia A at the same time.

Use of the pin hole 2a and the guide hole 9 enables inserting the hinge pin P1 to a predetermined position in the guide wire GW already inserted in a tibia A or enables inserting the guide wire GW to a predetermined position in the hinge pin P1 already inserted in a tibia A.

A center axis line K of the guide hole 9 is offset from a radial axis line J in a direction orthogonal to both the axis line I and the center axis line K. The radial axis line J is a radial axis line extending through the axis line I and is parallel to the center axis line K. An amount of offset of the center axis line K from the radial axis line J is designed so that a distance Δd becomes substantially equal to a thickness of an osteotome blade, preferably, the distance Δd becomes more than 0 mm and no more than 2 mm. The distance Δd is a distance in the offset direction from the axis line I to an outer circumferential surface of the guide wire GW extending through the guide hole 9. During osteotomization on an osteotomization line L, the osteotome blade is inserted along the outer circumferential surface of the guide wire GW. The guide wire GW being disposed at a position distant from the axis line I by the amount of the thickness of the osteotome blade enables making a center of rotation of osteotomization precisely coincide with a hinge point H.

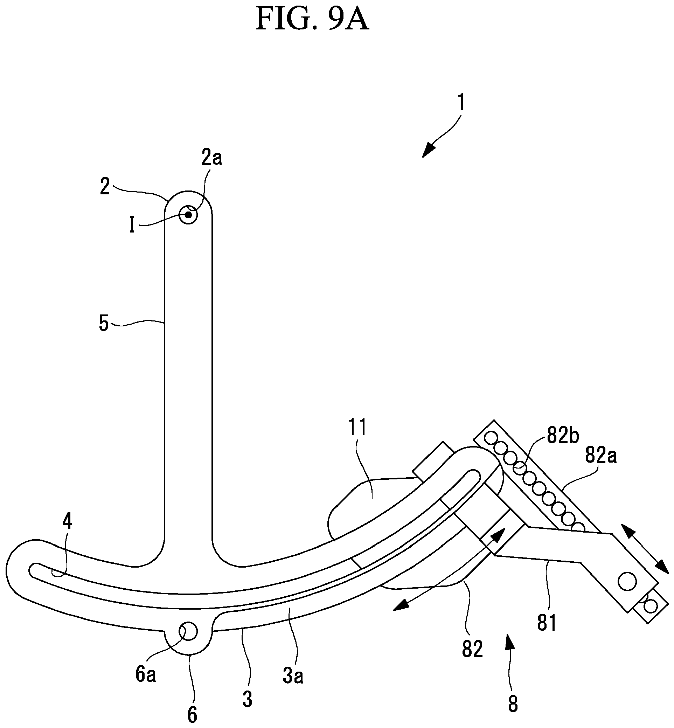

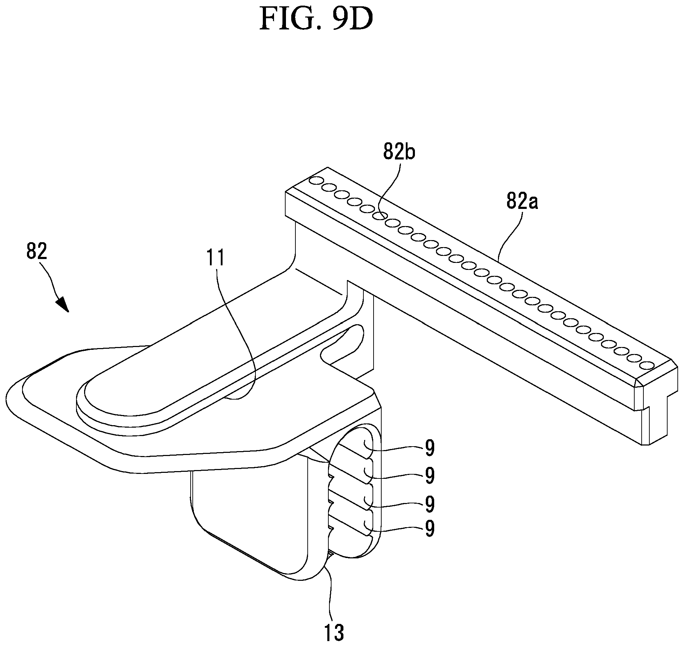

As illustrated in Figs. 9A to 9D, the second part 82 may further include a second guide portion 11 that guides the osteotome blade along a plane orthogonal to the axis line I during osteotomization for an undersurface E. The second guide portion 11 in Figs. 9A to 9C is a flat surface extending along a plane orthogonal to the axis line I. The second guide portion 11 in Fig. 9D is a flat slit extending along a plane orthogonal to the axis line I. The flat surface and slit each have a width that is larger than a width of the osteotome blade. In consideration of a positional relationship among the anterior surface of a tibia A, the osteotome blade and the guide wire GW, the second guide portion 11 is disposed between the guide portion 3 and the guide hole 9 in the up-down direction and extends from the guide hole 9 to the fixing portion 6 side. During osteotomization for an undersurface E, the osteotome blade being moved along the second guide portion 11 enables forming the undersurface E perpendicularly to the axis line I and the hinge pin P1. If the undersurface E is inclined relative to the hinge pin P1, one of a hinge portion and a tuberosity part B may become excessively thin. Provision of the second guide portion 11 enables prevention of such inconvenience.

The guide hole 9 and the second guide portion 11 may be movable in the up-down direction relative to the guide portion 3. For example, the second part 82 may include a first member joined to the first part 81 and a second member including the guide hole 9 and the second guide portion 11, and the second member may be joined to the first member in such a manner as to be movable in the up-down direction relative to the first member. This configuration enables adjusting a position of the undersurface E and a thickness of the tuberosity part B by adjusting a position of the second guide portion 11 in the direction parallel to the axis line I.

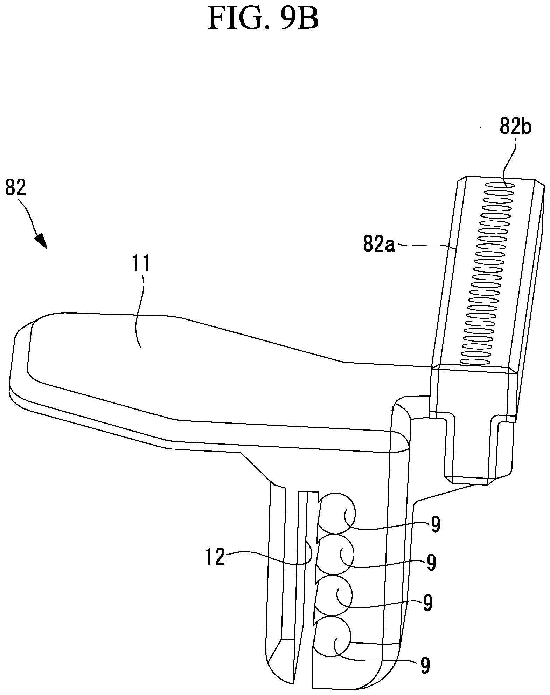

As illustrated in Fig. 9B, the second part 82 may include a flat guide slit 12 that is externally tangent to the guide hole 9 and extends in the up-down direction, as a third guide portion that guides the osteotome blade during osteotomization on an osteotomization line L. The osteotome blade being inserted to a tibia A through the guide slit 12 enables osteotomizing the tibia A in parallel with the guide wire GW with the bone surgery instrument 1 kept disposed on the tibia A.

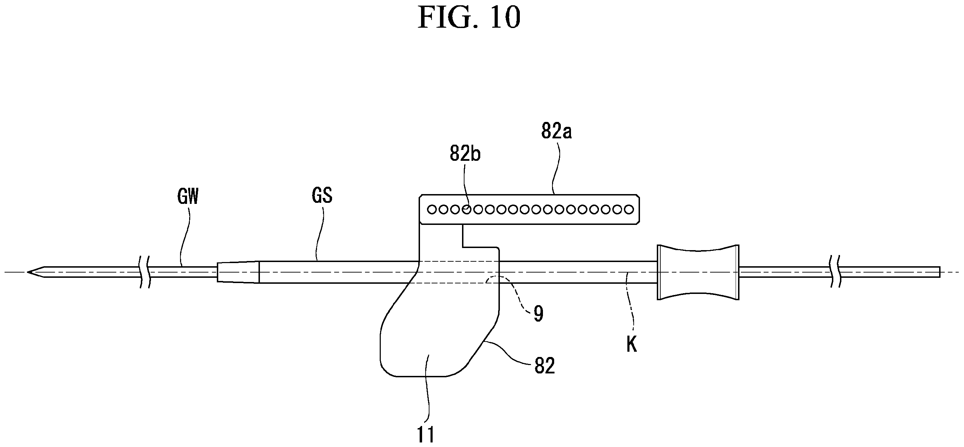

As illustrated in Fig. 10, when a guide wire GW is inserted to a tibia A, a circular tube-like guide sleeve GS to which a guide wire GW is inserted is sometimes used. Therefore, the guide hole 9 may have an inner diameter that is slightly larger than an outer diameter of the guide sleeve GS and guide the guide sleeve GS.

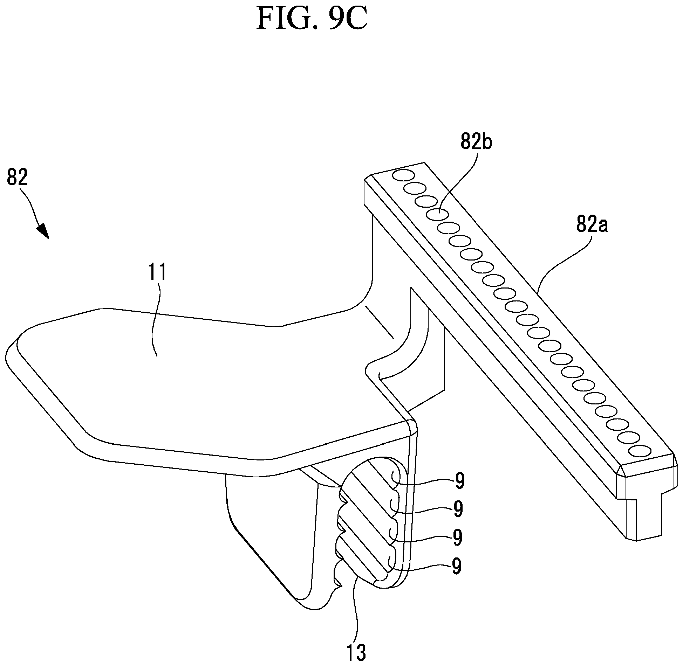

Where the guide hole 9 guides the guide sleeve GS, as illustrated in Figs. 9C and 9D, the second part 82 may have a slit 13 having a width that is larger than a diameter of the guide wire GW and extending in parallel with the guide hole 9.

The slit 13 is formed on the lower side of a guide hole 9 at a lowermost position, extends over an entire length of the guide hole 9 and brings the guide hole 9 into communication with the outside of the second part 82. Where a plurality of guide holes 9 are provided, all of the guide holes 9 communicate with one another in the up-down direction and a guide wire GW is movable between a guide hole 9 at an uppermost position and a guide hole 9 at a lowermost position in the up-down direction. Provision of the slit 13 enables removing the bone surgery instrument 1 from the tibia A from above, with the hinge pin P1 and the guide wire GW kept inserted in the tibia A. In other words, moving the entire bone surgery instrument 1 upward after the guide sleeve GS being removed from the guide hole 9 enables removing the guide wire GW from the guide hole 9 through the slit 13 and removing the hinge pin P1 from the pin hole 2a.

In Figs. 8A to 9D, the second part 82 is joined to the first part 81 in such a manner as to be linearly movable in a direction parallel to the center axis line K relative to the first part 81. In an example, the second part 82 includes a slider 82a extending in parallel with the center axis line K, and the slider 82a is supported by the first part 81 in such a manner as to be movable in a longitudinal direction thereof. Consequently, the second part 82 can be disposed at a position as close to a bone surface as possible, and with the second part 82 provided with no play, a guide wire GW can be inserted to a tibia A and osteotomization can be performed using an osteotome blade.

A positioning mechanism that positions the second part 82 relative to the first part 81 may be provided. For example, a ball plunger (illustration omitted) is provided in the first part 81 and a plurality of holes 82b arranged in a row, and the plurality of holes 82b each receiving the ball plunger, may be provided in the slider 82a. In order to temporarily fix the second part 82 to the first part 81 with greater force, a fixing member such as a fixing screw may further be provided.

The second part 82 may be fixed to the first part 81. In this case, the movable portion 8 does not necessarily need to be formed of two parts 81, 82 and may be formed of a single member.

### {Reference Signs List}

- 1, 10, 20: bone surgery instrument
- 2: center portion
- 2a: pin hole
- 3: guide portion
- 4: slit
- 5: support rod portion
- 6: fixing portion
- 6a: fixing hole
- 7: contact surface, concave surface
- 8: movable portion
- 9: guide hole
- 11: second guide portion
- A: tibia
- B: tuberosity part
- GW: guide wire
- H: hinge point (specific point)
- I: axis line
- P2: fixing pin (fixing member)

## Claims

1. A bone surgery instrument for guiding an osteotome blade in a circumferential direction around a specific point in a bone, the bone surgery instrument comprising:
a center portion to be positioned at the specific point;
a guide portion including a slit extending in a circumferential direction around an axis line extending through the center portion, the slit which is extending through the guide portion in a direction parallel to the axis line and which guides the osteotome blade inserted in the slit in the circumferential direction around the axis line; and
a fixing portion to be fixed to the bone using a fixing member.

2. The bone surgery instrument according to claim 1, wherein the bone surgery instrument is used for osteotomization in an arc-like or substantially arc-like shape at a distal portion of a tuberosity part of a tibia.

3. The bone surgery instrument according to claim 1 or 2, wherein the guide portion has a thickness that makes an amount of projection of a tip of the osteotome blade from the slit be equal to or below a predetermined amount.

4. The bone surgery instrument according to any of claims 1 to 3, wherein the center portion includes a pin hole extending through the center portion in a direction parallel to the axis line.

5. The bone surgery instrument according to any one of claims 1 to 4, wherein the fixing portion is provided at an opposite side of the center portion relative to the guide portion.

6. The bone surgery instrument according to any one of claims 1 to 5, wherein a width of the slit is larger than a thickness of the osteotome blade by 0.5 mm to 1.5 mm.

7. The bone surgery instrument according to any one of claims 1 to 6, wherein:
in a plan view seen along a direction of the axis line, an outer shape of the guide portion is a similar figure or a substantially similar figure of a shape of the slit; and
a thickness of a periphery portion of the guide portion, the periphery portion surrounding the slit, is 1 mm to 5 mm.

8. The bone surgery instrument according to any one of claims 1 to 7, comprising a contact surface on one side in a direction along the axis line, the contact surface being in contact with a surface of the bone,
wherein the contact surface is a concave surface adapted to a shape of the surface of the bone.

9. The bone surgery instrument according to any one of claims 1 to 8, comprising a support rod portion that interconnects the center portion and the guide portion,
wherein the support rod portion extends in a direction intersecting with the axis line, between the center portion and a center in the circumferential direction of the guide portion.

10. The bone surgery instrument according to any one of claims 1 to 9, wherein the slit extends along an arc having a constant curvature radius.

11. The bone surgery instrument according to any one of claims 1 to 9, wherein the slit extends along a curve having a curvature radius that gradually decreases in the circumferential direction around the axis line.

12. The bone surgery instrument according to claim 11, wherein the slit includes a plurality of linear portions that are continuous with each other in the circumferential direction around the axis line and each of the plurality of linear portions extends in a direction tangent or substantially tangent to a circle whose center is the axis line.

13. The bone surgery instrument according to claim 12, wherein:
each of triangles whose vertices are opposite ends of each of the linear portions and the center portion is a right triangle whose base is the linear portion; and
an opposite side of one of the right triangles and a hypotenuse of another one of the right triangles adjacent to the one of the right triangles are shared with each other.

14. The bone surgery instrument according to claim 11 or 12, wherein a distance between the center portion and the slit decreases by a predetermined length, for each position shifted by a predetermined angle in the circumferential direction around the axis line.

15. The bone surgery instrument according to any one of claims 1 to 14, wherein a distance between the center portion and the slit is 45 mm to 65 mm.

16. The bone surgery instrument according to any one of claims 4 to 15, comprising a movable portion joined to the guide portion in such a manner as to be movable in the circumferential direction around the axis line, wherein:
the movable portion includes a guide hole extending through the movable portion in a direction parallel to a radial direction orthogonal to the axis line and guiding, toward the axis line, a guide wire or a guide sleeve to which the guide wire is inserted; and
an outer circumferential surface of the guide wire extending through the guide hole is disposed at a position distant from the axis line in an offset direction which is orthogonal to the axis line and a center axis line of the guide hole.

17. The bone surgery instrument according to claim 16, wherein the movable portion includes a second guide portion that guides the osteotome blade along a plane orthogonal to the axis line.
